# EUROPEAN PATENT APPLICATION

(11) **EP 2 974 647 A1**
(43) Date of publication of application: **20.01.2016**
(21) Application number: 14803590.0
(22) Date of filing: 14.04.2014
(51) Int. Cl.: A61B 1/00

(54) **ENDOSCOPE**

(30) Priority: 29.05.2013 JP 2013113228
(71) Applicant: Olympus Corporation, Shibuya-ku Tokyo 151-0072 (JP)
(72) Inventor: IMAI Shigeru, Tokyo 151-0072 (JP); ADACHI Junichi, Tokyo 151-0072 (JP); TOMIYAMA Takahiro, Tokyo 151-0072 (JP)
(74) Representative: Schicker, Silvia
(86) International application number: PCT/JP2014/060627
(87) International publication number: WO 2014/192447

(57) **Abstract**

An endoscope 1 includes a traction member for bending driving 14 connected to a bending portion 3 and inserted through an insertion section 2, a bending operation section 5, 22, 23 that performs pulling and loosening operation of the traction member for bending driving 14 and performs bending operation of the entire bending portion 3, a first traction member for bending fixing 15 connected to the bending portion 3 and inserted through the insertion section 2, a first operation section for bending fixing 6a that performs pulling and loosening operation of the first traction member for bending fixing 15 and operates fixing or release of the entire bending portion 3, a second traction member for bending fixing 16 connected to the bending portion 3 and inserted through the insertion section 2; and a second operation section for bending fixing 6b that performs pulling and loosening operation of the second traction member for bending fixing 16 and operates fixing or release of a part of the bending portion 3.

## Description

### Technical Field

The present invention relates to an endoscope including a rigid insertion section in which a bending portion is provided.

### Background Art

In general, as endoscopes, there are a rigid endoscope configured by a rigid tube, an insertion section of which does not bend, and a flexible endoscope, an insertion section of which is configured by a flexible tube, including, at a distal end, a bending portion that can perform a bending action. The endoscopes are widely used in an abdominal cavity and a nose.

In the rigid endoscope, since the insertion section is rigid, hand operation is directly transmitted to a distal end. The distal end is easily controlled to a targeted position and a targeted direction. The rigid endoscope has an advantage that, for example, a visual field can be secured by pushing aside obstructive organs and tissues with the insertion section.

However, there is a problem in that the rigid endoscope can view only a front and, for example, every time an object present in an oblique position is observed, the rigid endoscope needs to be replaced with a rigid endoscope for oblique view having an exclusive visual field angle.

On the other hand, in the flexible endoscope, the insertion section is configured by a flexible tube. The flexible endoscope includes, on a distal end side of the insertion section, the bending portion that can be freely bent by operation of a hand operation section. Therefore, the flexible endoscope has an advantage that the flexible endoscope is excellent in insertability into an elongated conduit and the distal end can be bent in a desired viewing direction with a bending function.

In recent years, there has been a rigid endoscope with bending portion including both of the advantages of the rigid endoscope and the flexible endoscope. More specifically, the endoscope has a structure in which the bending portion of the flexible endoscope is provided in the insertion section at the distal end of the rigid endoscope and combined.

The bending portion provided in the conventional rigid endoscope is located between an insertion section configured by a rigid tube and a distal end portion of the insertion section. Several short joint rings are respectively connected in the bending portion by some means such as rivets or wires. A wire for driving is fixedly connected to a leading joint ring connected to the distal end portion.

Several or all of the following joint rings include, on insides, holes or exclusive routes for inserting the wire for driving. The wire for driving is inserted through the holes or the routes. When the wire for driving is pulled, the bending portion bends in a direction in which the wire for driving is pulled.

However, the conventional rigid endoscope with the bend includes a part that bends, i.e., the bending portion, compared with a rigid endoscope not including a bending mechanism. Therefore, there is a problem in that, for example, when the bending portion touches an inside of a body cavity, the distal end moves and a stable observation cannot be performed and a tissue cannot be pushed away in a vicinity of the distal end portion.

As measures against this problem, for example, Japanese Patent Publication No. 2010-207340 discloses a flexible bending guide tube. In the flexible bending guide tube, an end portion of a driving wire of a bending portion connected to a distal end of a hollow flexible tube is fixed to a slide member in an operation section. The slide member is capable of moving a member, which slides back and forth, back and forth by rotating an operation dial of the operation section. This makes it possible to perform a bending action by pulling the connected wire. Patent Literature 1 discloses a technique of a mechanism that can retain a shape of the bending tube while keeping the bending tube bent by fixing the slide member with hand operation.

However, in the conventional technique described in Japanese Patent Publication No. 2010-207340, with the mechanism, the entire bending portion is fixed at a time and, when the fixing of the bending portion is released, the fixing of the entire bending portion is released. Therefore, there is a problem in that a function of the fixing of the bending portion and a bending function cannot be allowed to coexist. That is, the conventional endoscope has a problem in that a visual field cannot be changed in a fixed state of the bending portion.

Therefore, in view of the circumstances, it is an object of the present invention to provide an endoscope with bending portion that can switch a bending portion to a state in which bending operation of the bending portion can be freely performed, a state in which the bending portion can be fixed, or a state in which bending operation of only a part of the bending portion can be performed and can allow a fixing function and a bending function of the bending portion to coexist.

### Disclosure of Invention

### Means for Solving the Problem

An endoscope according to an aspect of the present invention includes: an insertion section including a distal end portion and a bending portion; a traction member for bending driving connected to the bending portion and inserted through the insertion section; a bending operation section that performs pulling and loosening operation of the traction member for bending driving and performs bending operation of the entire bending portion; a first traction member for bending fixing connected to the bending portion and inserted through the insertion section; a first operation section for bending fixing that performs pulling and loosening operation of the first traction member for bending fixing and operates fixing or release of the entire bending portion; a second traction member for bending fixing connected to the bending portion and inserted through the insertion section; and a second operation section for bending fixing that performs pulling and loosening operation of the second traction member for bending fixing and operates fixing or release of a part of the bending portion.

According to the present invention described above, it is possible to provide an endoscope with bending portion that can switch a bending portion to a state in which bending operation of the bending portion can be freely performed, a state in which the bending portion can be fixed, or a state in which bending operation of only a part of the bending portion can be performed and can allow a fixing function and a bending function of the bending portion to coexist.

### Brief Description of the Drawings

Fig. 1 is an overall configuration diagram of a rigid endoscope in a first embodiment;
Fig. 2 is a sectional view showing an internal structure of a bending portion of an insertion section in the first embodiment;
Fig. 3 is a sectional view of the bending portion taken along line III-III in Fig. 2 in the first embodiment;
Fig. 4 is a sectional view of the bending portion taken along line IV-IV in Fig. 2 in the first embodiment;
Fig. 5 is a sectional view of the bending portion taken along line V-V in Fig. 2 in the first embodiment;
Fig. 6 is a sectional view of the bending portion taken along line VI-VI in Fig. 2 in the first embodiment;
Fig. 7 is an enlarged view of a part surrounded by a broken line circle VII in Fig. 4 in the first embodiment;
Fig. 8 is an enlarged view of a part surrounded by a broken line circle VIII in Fig. 5 in the first embodiment;
Fig. 9 is a sectional view showing a configuration inside an operation section in the first embodiment;
Fig. 10 is a perspective view showing a mechanism around a drum unit in the first embodiment;
Fig. 11 is a schematic diagram for explaining a bending action of the bending portion in the first embodiment;
Fig. 12 is a sectional view showing a configuration inside the operation section in a state in which an entire-bending fixing knob and a proximal-end-side-joint-ring-group fixing knob are operated to a proximal end side in the first embodiment;
Fig. 13 is a sectional view showing a configuration inside the operation section in a state in which the entire-bending fixing knob is operated to the proximal end side and the proximal-end-side-joint-ring-group fixing knob is operated to a distal end side in the first embodiment;
Fig. 14 is a sectional view showing a configuration inside the operation section in a state in which the entire-bending fixing knob is operated to the distal end side and the proximal-end-side-joint-ring-group fixing knob is operated to the proximal end side in the first embodiment;
Fig. 15 is a sectional view showing an internal structure of a bending portion of an insertion section in a second embodiment;
Fig. 16 is a sectional view of the bending portion taken along line XVI-XVI in Fig. 15 in the second embodiment;
Fig. 17 is a sectional view of the bending portion taken along line XVII-XVII in Fig. 15 in the second embodiment;
Fig. 18 is a sectional view of the bending portion taken along line XVIII-XVIII in Fig. 15 in the second embodiment;
Fig. 19 is a sectional view of the bending portion taken along line XVI-XVI in Fig. 15 of the second embodiment;
Fig. 20 is an enlarged view of a part surrounded by a broken line circle XX in Fig. 18 in the second embodiment;
Fig. 21 is an enlarged view of a part surrounded by a broken line circle XXI in Fig. 19 in the second embodiment;
Fig. 22 is a sectional view showing a configuration inside an operation section in the second embodiment;
Fig. 23 is a sectional view showing a configuration inside the operation section in a state in which an entire-bending fixing knob is operated to a proximal end side and a proximal-end-side-joint-ring-group fixing knob is operated to a distal end side in the second embodiment; and
Fig. 24 is a sectional view showing a configuration inside the operation section in a state in which the entire-bending fixing knob is operated to the distal end side and the proximal-end-side-joint-ring-group fixing knob is operated to the proximal end side in the second embodiment.

### Best Mode for Carrying Out the Invention

A rigid endoscope of the present invention is explained below with reference to the drawings. Note that, in the following explanation, the drawings based on embodiments are schematic and relations between thicknesses and widths of respective portions, ratios of the thicknesses of the respective portions, and the like are different from real ones. Portions having different relations and ratios of dimensions are sometimes included among the drawings.

### (First Embodiment)

First, a first embodiment of the present invention is explained.

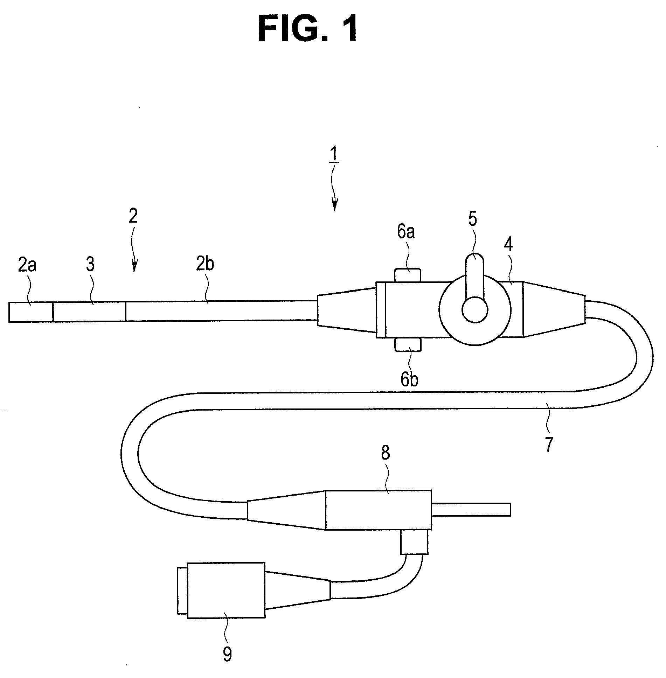

As shown in Fig. 1, a rigid endoscope 1 functioning as an endoscope with bending portion in the present embodiment includes an elongated insertion section 2 for insertion into a subject and an operation section 4 connected to a proximal end of the insertion section 2. A universal cord 7 extends from the operation section 4. A light guide connector 8 connected to a not-shown light source device is disposed at a proximal end of the universal cord 7.

A video connector 9 extends from the light guide connector 8. The video connector 9 is connected to a not-shown video processor.

In the elongated insertion section 2 inserted into the subject, a distal end portion 2a and a bending portion 3 are provided in a distal end part. The insertion section 2 includes a rigid tube 2b on a proximal end side of the bending portion 3.

In the operation section 4, a bending operation lever 5 for performing bending operation of the bending portion 3 and a bending-portion-entire-bending fixing knob 6a and a proximal-end-side-joint-ring-group fixing knob 6b functioning as operation section for bending fixing (operation means for bending fixing) for fixing a bent state of the bending portion 3 are provided. Note that specific configurations of the bending operation lever 5, the bending-portion-entire-bending fixing knob 6a, and the proximal-end-side-joint-ring-group fixing knob 6b are explained below.

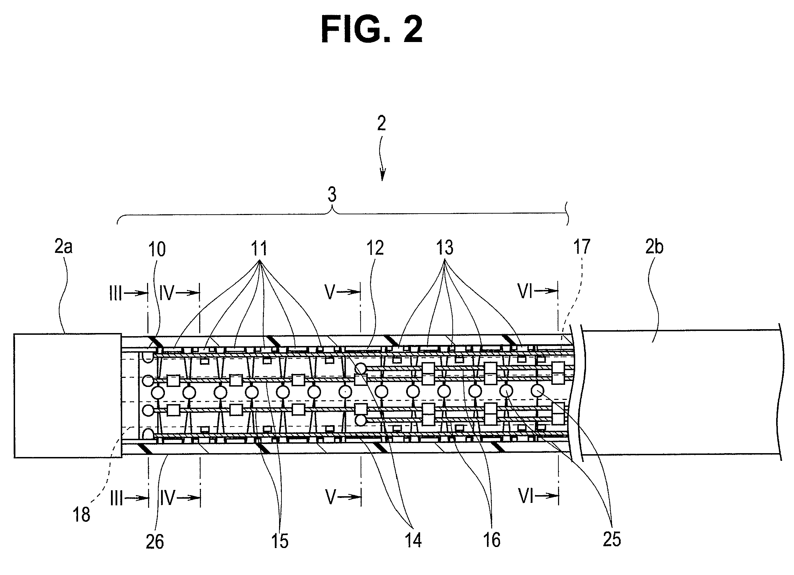

Light inputted from the light source device via the light guide connector 8 is transmitted by light guide cables 17 (see Fig. 2) inserted through an inside of the rigid endoscope 1 and irradiated on the subject via a not-shown lens disposed in the distal end portion 2a of the rigid endoscope 1.

The rigid endoscope 1 has a mechanism for transmitting an image captured by a camera unit disposed in the distal end portion 2a through a video unit cable 18 (see Fig. 2) inserted through the inside, transmitting the image to the video processor via the video connector 9, and sending the image to a not-shown monitor, and projecting the image.

The bending portion 3 of the rigid endoscope 1 is rotatably formed with a plurality of joint rings 11, 12, and 13 connected by rivets 25 as shown in Fig. 2. More specifically, the bending portion 3 includes a distal-end-side joint ring group 11 and a proximal-end-side joint ring group 13. A joint ring for relay 12 is provided between the distal-end-side joint ring group 11 and the proximal-end-side joint ring group 13.

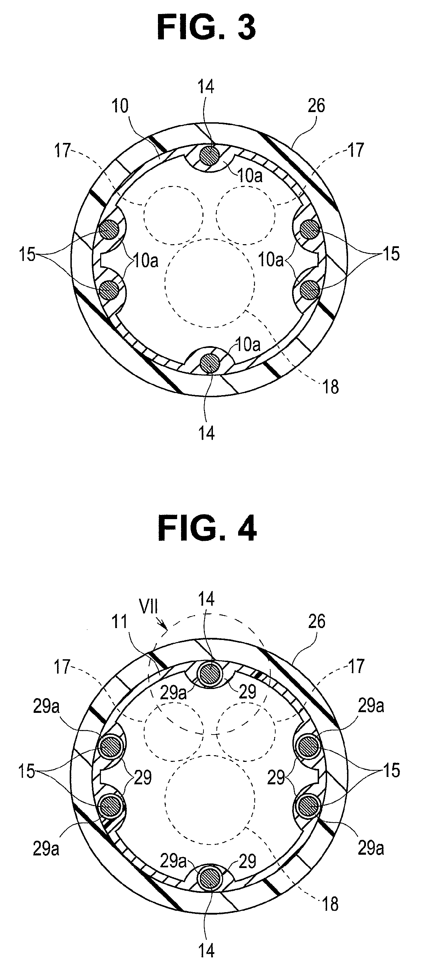

In a bending-tube first joint ring 10 present at a top of the distal-end-side joint ring group 11, as shown in Fig. 3, at least two bending operation wires 14 are connected to wire fixing sections 10a. Note that the at least two bending operation wires 14 are desirably disposed in opposed positions but do not have to be disposed in the opposed positions.

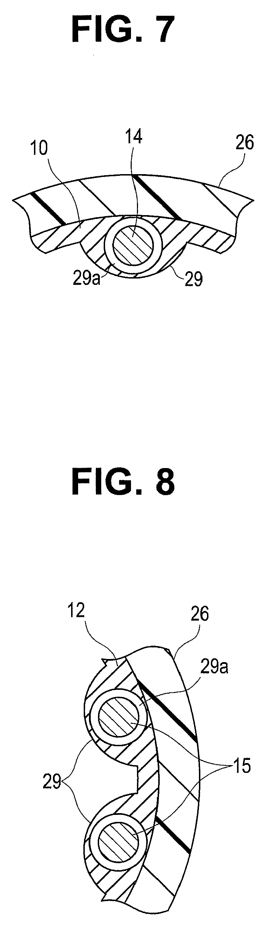

For example, as shown in Fig. 4 and Fig. 7, the two bending operation wires 14 are extended to an inside of the operation section 4 through wire guide routes 29a formed in wire guides 29 provided in the distal-end-side joint ring group 11, the joint ring for relay 12, and the proximal-end-side joint ring group 13.

Note that, in the rigid endoscope 1 in the present embodiment, the bending portion 3 having two bending directions is explained as an example. However, bending directions of the bending portion 3 is not limited to the two directions. The bending portion 3 may have more bending directions. In this case, with a center of the bending portion 3 set as an axis, at least one bending operation wire 14 is set in each of a desired bending direction and an opposite side of the direction.

However, a shift of a setting position of the bending operation wires 14 with respect to the desired bending direction can be allowed.

As shown in Fig. 3, the bending-tube first joint ring 10 of the bending portion 3 includes a plurality of wire fixing sections 10a. At least two or more, here, four bending-portion entire fixing wires 15 are fixedly connected to the bending-tube first joint ring 10 by the wire fixing sections 10a. Fixing positions of the bending-portion entire fixing wires 15 to the bending-tube first joint ring 10 are desirably arranged in opposed positions but do not always have to be opposed positions.

As shown in Fig. 4 to Fig. 6 and Fig. 8, the bending-portion entire fixing wires 15 are inserted through the wire guide routes 29a drilled in the wire guides 29 of the distal-end-side joint ring group 11 and the proximal-end-side joint ring group 13.

Note that the wire guide routes 29a do not always have to be present in all the joint rings. Positions of the wire guide routes 29a are desirably opposed positions but do not always have to be the opposed positions.

The bending-portion entire fixing wires 15 are extended to the inside of the operation section 4 through an inside of the insertion section 2.

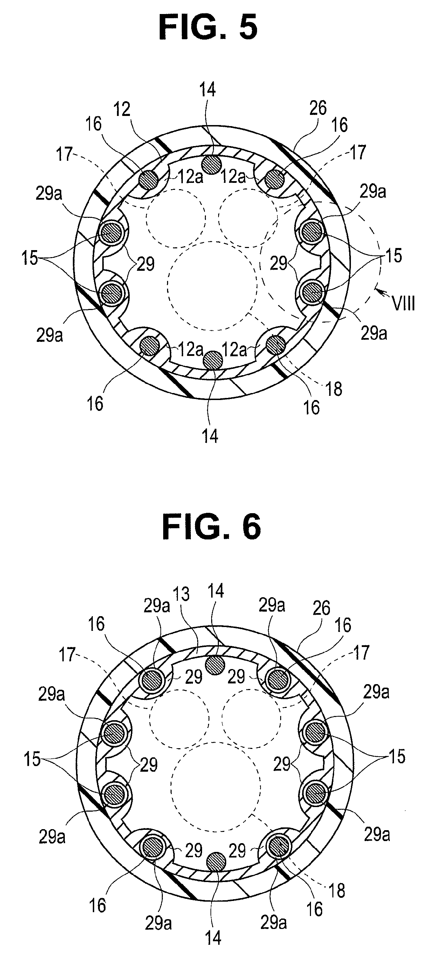

As shown in Fig. 5, the joint ring for relay 12 of the bending portion 3 includes a plurality of wire fixing sections 12a. At least two, here, four proximal-end-side-joint-ring-group fixing wires 16 are fixedly connected to the wire fixing sections 12a.

Note that fixing positions of the proximal-end-side-joint-ring-group fixing wires 16 to the joint ring for relay 12 are desirably arranged in positions opposed to one another but do not always have to be the opposed positions.

As shown in Fig. 6, the proximal-end-side-joint-ring-group fixing wires 16 are inserted through the wire guide routes 29a drilled in the wire guides 29 of the proximal-end-side joint ring group 12 and extended to the inside of the operation section 4 through the inside of the insertion section 2.

Note that the bending portion 3 is covered with bending rubber 26 on an outer side and has a structure for preventing intrusion of foreign matters, liquid, and the like into a movable section.

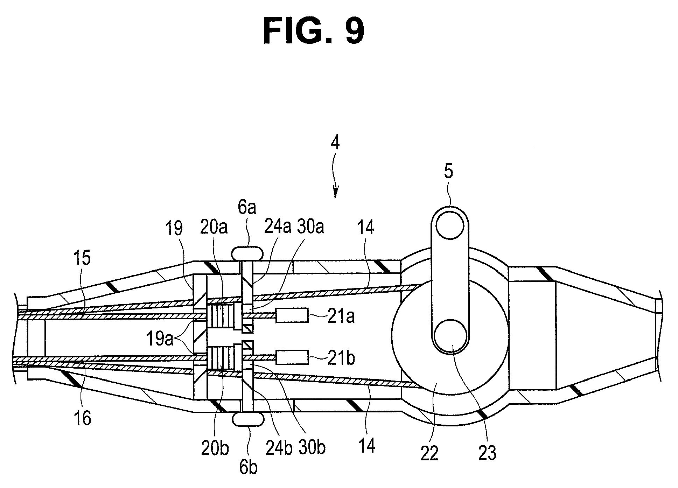

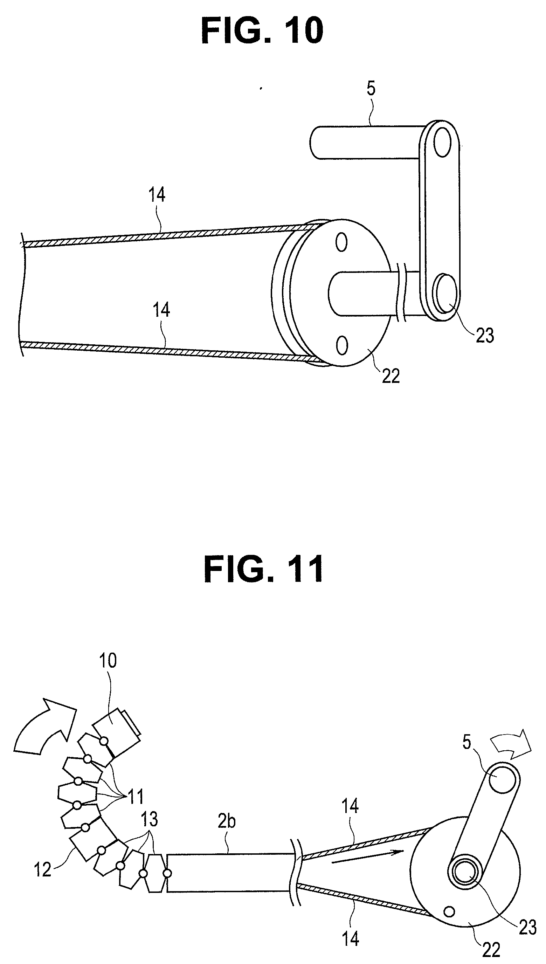

As shown in Fig. 9 and Fig. 10, the bending operation wires 14 drawn into the inside of the operation section 4 from the insertion section 2 are fixedly connected to a part of an arcuate surface of a drum unit 22 provided on the inside of the operation section 4. Note that the bending operation wires 14 configure a traction member for bending driving.

That is, as shown in Fig. 2 and Fig. 3, the two bending operation wires 14 are formed in a pair in a state in which distal end portions thereof are fixed in upper and lower opposed positions in the bending-tube first joint ring 10. Respective proximal end portions of the two bending operation wires 14 formed in the pair in this way are assembled to opposed positions in a part of an outer circumference of the drum unit 22 as on an inside of the bending-tube first joint ring 10.

A drum shaft 23 is fixed to the center of the drum unit 22. Note that the drum shaft 23 has to be fixedly connected to the drum unit 22. A fixing position of the drum shaft 23 does not have to be a center of the drum unit 22.

The drum shaft 23 is rotatably attached to an inner surface of the operation section 4. The drum shaft 23 has to be not firmly fixed to the operation section 4 and can freely rotate.

Note that the drum shaft 23 pierces through an outer wall of the operation section 4. The bending operation lever 5 is fixedly connected to the drum shaft 23. Alternatively, the bending operation lever 5 pierces through the outer wall of the operation section 4 and fixedly connected to the drum shaft 23 on the inside.

The bending operation lever 5 can be moved to turn around an axis of the drum shaft 23. The drum unit 22 on the inside is turned in association with an action of the bending operation lever 5.

For example, as shown in Fig. 11, the drum unit 22 can be turned around the axis of the coupled drum shaft 23 by moving the bending operation lever 5 to a rear side of the rigid endoscope 1. By turning the drum unit 22 by turning operation of the bending operation lever 5 in this way, one of the pair of bending operation wires 14 coupled to the drum unit 22 is wound and pulled by the drum unit 22. The other of the pair is pushed out and loosened.

According to such an operation, one of the bending operation wires 14 is pulled to be drawn in a direction of the operation section 4. The other of the bending operation wires 14 is pushed out in a direction of the insertion section 2. Consequently, the bending-tube first joint ring 10 is pulled to a side where a distal end of one bending operation wire 14 is fixed.

According to this series of operation, here, the bending portion 3 is operated to bend at an intended angle in upward and downward two directions. In this way, to perform the bending operation of the bending portion 3, a bending operation section (bending operation means) for performing traction operation of the pair of bending operation wires 14 is configured.

Referring back to Fig. 9, a bending fixing wire guide 19 is fixedly set inside the operation section 4. In the bending fixing wire guide 19, a plurality of holes 19a having size enough for allowing the four bending-portion entire fixing wires 15 and the four proximal-end-side-joint-ring-group fixing wires 16 functioning as the traction member for bending fixing to smoothly pass are formed (note that, in Fig. 9, only two holes 19a are shown).

That is, the bending-portion entire fixing wires 15 and the proximal-end-side-joint-ring-group fixing wires 16 drawn from the insertion section 2 are inserted through the plurality of holes 19a formed in the bending fixing wire guide 19 one by one. Note that the holes 19a and the bending-portion entire fixing wires 15 and the proximal-end-side-joint-ring-group fixing wires 16 may be in contact with each other.

After passing the holes 19a of the bending fixing wire guide 19, the bending-portion entire fixing wires 15 and the proximal-end-side-joint-ring-group fixing wires 16 are inserted through springs for wire fixing 20a and 20b one by one.

One ends of the springs for wire fixing 20a and 20b are in contact with a proximal end face of the bending fixing wire guide 19. Note that end portions of the springs for wire fixing 20a and 20b may be fixed to the bending fixing wire guide 19 or may be simply in contact with the bending fixing wire guide 19.

An outer diameter of spring for wire fixing 20a and 20b is set larger than a diameter of the holes 19a of the bending fixing wire guide 19.

The bending-portion entire fixing wires 15 inserted through the spring for wire fixing 20a are subsequently inserted through stopper release hole 30a formed in an entire-bending-fixing spring holder 24a one by one. Note that a hole diameter of the stopper release hole 30a drilled in the entire-bending-fixing spring holder 24a is set smaller than an outer diameter of the spring for wire fixing 20a.

A stopper for wire fixing 21 a is fixedly assembled to end portions of the bending-portion entire fixing wires 15 inserted through the stopper release hole 30a. An outer diameter of the stopper for wire fixing 21a is set smaller than the hole diameter of the stopper release hole 30a formed in the entire-bending-fixing spring holder 24a. The outer diameter of the stopper for wire fixing 21 a is set larger than an inner diameter of the spring for wire fixing 20a.

The spring for wire fixing 20a is set to a dimension in a longitudinal direction with which, when one side of the spring for wire fixing 20a comes into contact with a surface on a proximal end side of the bending fixing wire guide 19 and the spring for wire fixing 20a is released to an free length on the basis of a position of the contact, the spring for wire fixing 20a comes into contact with the stopper for wire fixing 21 a and can push the stopper for wire fixing 21 a with a predetermined force.

An assembling position of the stopper for wire fixing 21 a connected to the bending-portion entire fixing wires 15 is set to a position where, in a state in which the spring for wire fixing 20a is released to the free length, the bending-portion entire fixing wires 15 in contact with the spring for wire fixing 20a and urged with the predetermined force receive tension to the proximal end side.

The entire-bending-fixing spring holder 24a is fixedly connected to the entire-bending fixing knob 6a located on an outer surface of the operation section 4. The entire-bending fixing knob 6a is enabled to operate a position of the entire-bending-fixing spring holder 24a on the inside of the operation section 4 back and forth from the outside.

According to the operation of the entire-bending fixing knob 6a, it is possible to move the entire-bending-fixing spring holder 24 connected to the entire-bending fixing knob 6a to the distal end side and compress the spring for wire fixing 20a. Note that, in the operation section 4, a not-shown fixing mechanism for locking the entire-bending fixing knob 6a in a position where the spring for wire fixing 20a is compressed is provided.

On the other hand, the proximal-end-side-joint-ring-group fixing wires 16 inserted through the spring for wire fixing 20b are subsequently inserted through stopper release hole 30b formed in a proximal-end-side-joint-ring-group-fixing spring holder 24b one by one. The stopper release hole 30b drilled in the proximal-end-side-joint-ring-group-fixing spring holder 28b is set smaller than an outer diameter of the spring for wire fixing 20b.

A stopper for wire fixing 21 b is fixedly assembled to end portions of the proximal-end-side-joint-ring-group fixing wires 16 inserted through the stopper release hole 30b formed in the proximal-end-side-joint-ring-group-fixing spring holder 28b. An outer diameter of the stopper for wire fixing 21b is set smaller than the stopper release holes 30b opened in the proximal-end-side-joint-ring-group-fixing spring holder 24b.

An assembling position of the stopper for wire fixing 21b connected to the proximal-end-side-joint-ring-group fixing wires 16 is set to positions where, in a state in which the spring for wire fixing 20b is released to the free length, the proximal-end-side-joint-ring-group fixing wires 16 in contact with the spring for wire fixing 20b and urged with the predetermined force receive tension to the proximal end side.

The proximal-end-side-joint-ring-group-fixing spring holder 24b is fixedly connected to the proximal-end-side-joint-ring-group fixing knob 6b located on the outer surface of the operation section 4. By moving the proximal-end-side-joint-ring-group fixing knob 6b, a position of the proximal-end-side-joint-ring-group-fixing spring holder 24b on the inside of the operation section 4 can be operated from the outside.

By operating the proximal-end-side-joint-ring-group fixing knob 6b, it is possible to move the proximal-end-side-joint-ring-group-fixing spring holder 24b connected to the proximal-end-side-joint-ring-group fixing knob 6b to the distal end side and compress the spring for wire fixing 20b. Note that, in the operation section 4, a not-shown fixing mechanism for locking the proximal-end-side-joint-ring-group fixing knob 6b in a position where the spring for wire fixing 20b is compressed is provided.

In the rigid endoscope 1 in the present embodiment configured as explained above, for example, in a state shown in Fig. 9, the entire-bending fixing knob 6a and the proximal-end-side-joint-ring-group fixing knob 6b are in an operation position where the entire-bending fixing knob 6a and the proximal-end-side-joint-ring-group fixing knob 6b are moved to the distal end side. At this point, the entire-bending fixing knob 6a and the proximal-end-side-joint-ring-group fixing knob 6b are fixed in operated positions by the lock mechanism provided in the operation section 4.

In this state, the entire-bending-fixing spring holder 24a and the proximal-end-side-joint-ring-group-fixing spring holder 24b coupled to the entire-bending fixing knob 6a and the proximal-end-side-joint-ring-group fixing knob 6b compress all of the springs for wire fixing 20a and 20b to the distal end side.

In this state, the springs for wire fixing 20a and 20b are not in contact with the stoppers for wire fixing 21a and 21b. The stoppers for wire fixing 21a and 21b can freely move back and forth.

That is, in the operation position where the entire-bending fixing knob 6a and the proximal-end-side-joint-ring-group fixing knob 6b are moved to the distal end side shown in Fig. 9, the bending-portion entire fixing wires 15 and the proximal-end-side-joint-ring-group fixing wires 16, to which the stoppers for wire fixing 21a and 21b are connected, can freely move back and forth.

Therefore, the bending-tube first joint ring 10, the distal-end-side joint ring group 11, the joint ring for relay 12, and the proximal-end-side joint ring group 13 do not receive a traction force to the proximal end side from the bending-portion entire fixing wires 15 and the proximal-end-side-joint-ring-group fixing wires 16 and can freely turn in respective coupling parts thereof.

Consequently, by operating the bending operation lever 5, it is possible to freely perform bending operation of the bending portion 3 according to pulling and loosening of the pair of bending operation wires 14 coupled to the drum unit 22 associated with the bending operation lever 5.

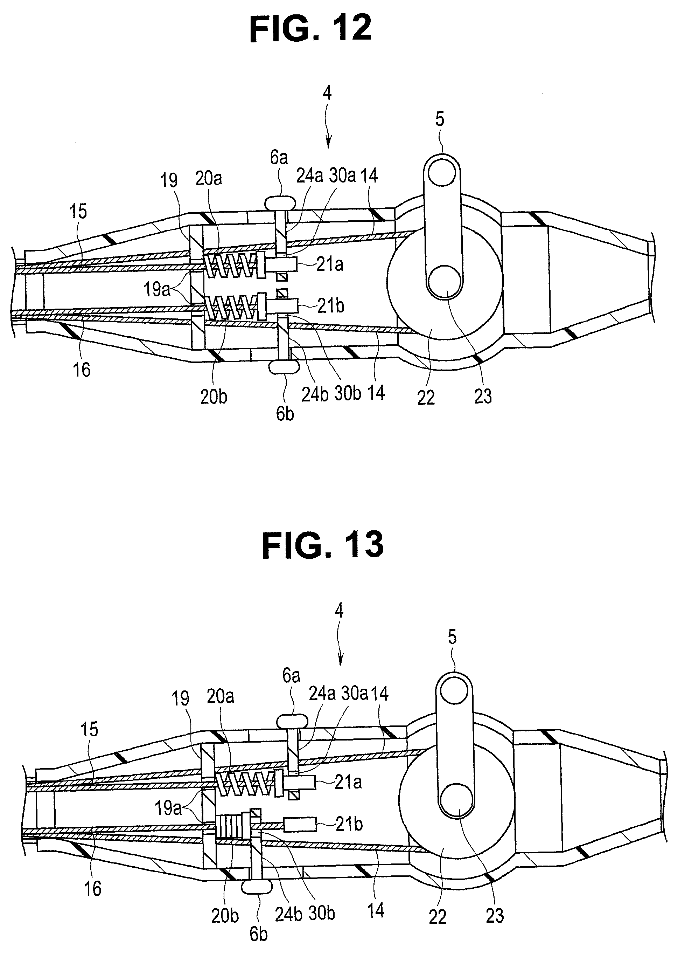

As shown in Fig. 12, for example, in an operation position where both of the entire-bending fixing knob 6a and the proximal-end-side-joint-ring-group fixing knob 6b are moved to the proximal end side, the entire-bending-fixing spring holder 24a and the proximal-end-side-joint-ring-group-fixing spring holder 24b coupled to the entire-bending fixing knob 6a and the proximal-end-side-joint-ring-group fixing knob 6b do not come into contact with the springs for wire fixing 20a and 20b. The compression of the springs for wire fixing 20a and 20b is released.

At this point, the stoppers for wire fixing 21a and 21b pass the stopper release holes 30a and 30b provided in the entire-bending-fixing spring holder 24a and the proximal-end-side-joint-ring-group-fixing spring holder 24b.

One end on the distal end side of each of the springs for wire fixing 20a and 20b, the compression of which is released, presses the bending fixing wire guide 19. However, since the bending fixing wire guide 19 is fixed and provided on the inside of the operation section 4, the springs for wire fixing 20a and 20b press the stoppers for wire fixing 21a and 21b in contact with the springs for wire fixing 20a and 20b to the proximal end side.

Consequently, all of the bending-portion entire fixing wires 15 and the proximal-end-side-joint-ring-group fixing wires 16, to which the stoppers for wire fixing 21a and 21 b are connected, are attracted and pulled to the proximal end side by urging forces of the springs for wire fixing 20a and 20b. That is, the springs for wire fixing 20a and 20b configure a traction member for bending portion fixing that pulls the bending-portion entire fixing wires 15 and the proximal-end-side-joint-ring-group fixing wires 16.

In this state, the bending-tube first joint ring 10, the distal-end-side joint ring group 11, the joint ring for relay 12, and the proximal-end-side joint ring group 13 receive a traction force to the proximal end side by the bending-portion entire fixing wires 15 and the proximal-end-side-joint-ring-group fixing wires 16. All of the bending-tube first joint ring 10, the distal-end-side joint ring group 11, the joint ring for relay 12, and the proximal-end-side joint ring group 13 are drawn to the proximal end side.

That is, the bending-tube first joint ring 10, the distal-end-side joint ring group 11, the joint ring for relay 12, and the proximal-end-side joint ring group 13 are compressed to the proximal end side. Mobility in respective coupling parts of the bending-tube first joint ring 10, the distal-end-side joint ring group 11, the joint ring for relay 12, and the proximal-end-side joint ring group 13 is suppressed.

As a result, movement of the pair of bending operation wires 14 is also suppressed. The bending operation lever 5 that pulls and loosens the bending operation wires 14 cannot be freely moved, operation of the bending operation lever 5 is suppressed, and bending operation of the bending portion 3 cannot be freely performed.

Note that, at this point, the bending portion 3 is in a fixed state in which the bending portion 3 is substantially linear and not bent because the bending-tube first joint ring 10, the distal-end-side joint ring group 11, the joint ring for relay 12, and the proximal-end-side joint ring group 13 receive a uniform traction force to the proximal end side in the circumferential direction by the bending-portion entire fixing wires 15 and the proximal-end-side-joint-ring-group fixing wires 16. That is, the insertion section 2 is fixed in a substantially linear shape.

However, when a force exceeding the urging force of the springs for wire fixing 20a and 20b is directly applied to the bending portion 3 from the outside or when the force is applied to the bending operation lever 5, the bending portion 3 can bend.

As shown in Fig. 13, for example, in an operation position where the entire-bending fixing knob 6a is moved to the proximal end side and the proximal-end-side-joint-ring-group fixing knob 6b is moved to the distal end side, only the entire-bending-fixing spring holder 24a coupled to the entire-bending fixing knob 6a releases the compression of the spring for wire fixing 20a.

At this point, the stopper for wire fixing 21a passes the stopper release hole 30a provided in the entire-bending-fixing spring holder 24a.

As explained above, one end on the distal end side of the spring for wire fixing 20a, the compression of which is released, presses the bending fixing wire guide 19 and presses the stopper for wire fixing 21a in contact with the spring for wire fixing 20a to the proximal end side. Consequently, all of the bending-portion entire fixing wires 15, to which the stopper for wire fixing 21 a is coupled, are attracted and pulled to the proximal end side by an urging force of the spring for wire fixing 20a.

In this state, as explained above, all of the bending-tube first joint ring 10, the distal-end-side joint ring group 11, the joint ring for relay 12, and the proximal-end-side joint ring group 13 receive a traction force to the proximal end side by the bending-portion entire fixing wires 15 and drawn to the proximal end side.

That is, as explained above, the bending-tube first joint ring 10, the distal-end-side joint ring group 11, the joint ring for relay 12, and the proximal-end-side joint ring group 13 are compressed to the proximal end side. Mobility in the respective coupling parts is suppressed. As a result, movement of the pair of bending operation wires 14 is also suppressed. The bending operation lever 5 that pulls and loosens the bending operation wires 14 cannot be freely moved, operation of the bending operation lever 5 is suppressed, and bending operation of the bending portion 3 cannot be freely performed.

Note that, at this point as well, the bending portion 3 is in a fixed state in which the bending portion 3 is substantially linear and not bent because the bending-tube first joint ring 10, the distal-end-side joint ring group 11, the joint ring for relay 12, and the proximal-end-side joint ring group 13 inside the bending portion 3 receive a uniform traction force to the proximal end side in the circumferential direction by the bending-portion entire fixing wires 15 and the proximal-end-side-joint-ring-group fixing wires 16. That is, the insertion section 2 is fixed in a substantially linear shape.

However, when a force exceeding the urging force of the spring for wire fixing 20a is directly applied to the bending portion 3 from the outside or when the force is applied to the bending operation lever 5, the bending portion 3 can bend.

Note that, since the bending portion 3 does not receive an urging force of the spring for wire fixing 20b, if the force exceeding the urging force of only the spring for wire fixing 20a is applied to the bending portion 3, the bending portion 3 can bend.

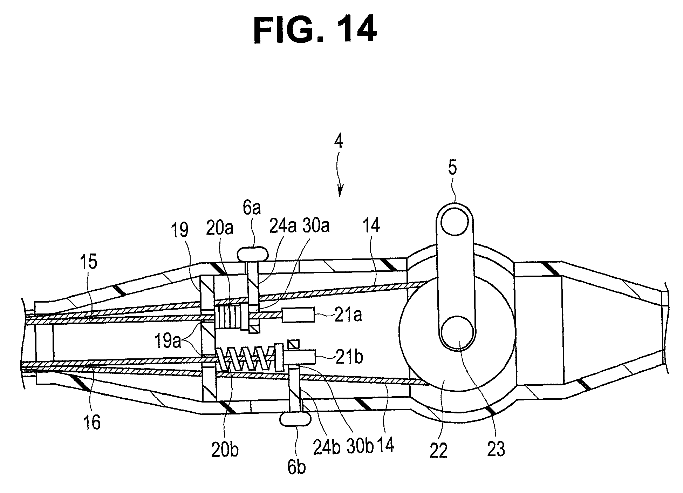

As shown in Fig. 14, for example, in an operation position where the entire-bending fixing knob 6a is moved to the distal end side and the proximal-end-side-joint-ring-group fixing knob 6b is moved to the proximal end side, only the proximal-end-side-joint-ring-group-fixing spring holder 24b coupled to the proximal-end-side-joint-ring-group fixing knob 6b releases the compression of the spring for wire fixing 20b.

At this point, the stopper for wire fixing 21b passes the stopper release hole 30b provided in the proximal-end-side-joint-ring-group-fixing spring holder 24b.

As explained above, one end on the distal end side of the spring for wire fixing 20b, the compression of which is released, presses the bending fixing wire guide 19 and presses the stopper for wire fixing 21 b in contact with the spring for wire fixing 20b to the proximal end side.

Consequently, all of the proximal-end-side-joint-ring-group fixing wires 16, to which the stopper for wire fixing 21b is coupled, are attracted and pulled to the proximal end side by an urging force of the spring for wire fixing 20b.

In this state, only the joint ring for relay 12, to which distal ends of the proximal-end-side-joint-ring-group fixing wires 16 are fixed, and the proximal-end-side joint ring group 13 on the proximal end side of the joint ring for relay 12 receive a traction force to the proximal end side by the proximal-end-side-joint-ring-group fixing wires 16 and drawn to the proximal end side.

That is, the joint ring for relay 12 and the proximal-end-side joint ring group 13 are compressed to the proximal end side. Mobility in respective coupling parts of the joint ring for relay 12 and the proximal-end-side joint ring group 13 is suppressed. The bending portion 3 is in a fixed state in which the bending portion 3 is substantially linear and not bent because the joint ring for relay 12 and the proximal-end-side joint ring group 13 receive a uniform traction force to the proximal end side in the circumferential direction by and the proximal-end-side-joint-ring-group fixing wires 16. Therefore, only a part where the joint ring for relay 12 and the proximal end side joint ring group 13 are provided is in a fixed state in which the part is substantially linear and not bent.

In the bending-tube first joint ring 10 and the distal-end-side joint ring group 11 that do not receive the traction force to the proximal end side by the proximal-end-side-joint-ring-group fixing wires 16, mobility in the respective coupling parts is not suppressed. The bending-tube first joint ring 10 and the distal-end-side joint ring group 11 can turn freely.

Therefore, by operating the bending operation lever 5, it is possible to perform bending operation of a part where the bending-tube first joint ring 10 and the distal-end-side joint ring group 11 are provided on the distal end side of the bending portion 3.

Note that, as explained above, when a force exceeding the urging force of the spring for wire fixing 20b is directly applied from the outside to the part where the joint ring for relay 12 and the proximal-end-side joint ring group 13 are provided on the proximal end side of the bending portion 3 or when the force is applied to the bending operation lever 5, the entire bending portion 3 can bend.

As explained above, the rigid endoscope 1 in the present embodiment is configured to be capable of selectively taking, by operating back and forth the entire-bending fixing knob 6a and the proximal-end-side-joint-ring-group fixing knob 6b provided in the operation section 4, a state in which bending operation of the bending portion 3 can be freely performed, a state in which the bending portion 3 can be substantially linearly fixed, or a state in which bending operation of only the distal end side of the bending portion 3 can be performed and the proximal end side can be substantially linearly fixed.

That is, the rigid endoscope 1 can be configured to be capable of selecting preservation of a bending function only in a part of the bending portion 3, that is, the proximal end side while hardening the entire insertion section 2 or hardening a part of the bending portion 3.

By adopting such a configuration, the rigid endoscope 1 is a rigid endoscope with bending portion that can eliminate labor for replacing a front-view endoscope and a perspective-view endoscope, reduce an observation time, and perform an observation in a range wider than an observation range of the conventional endoscope.

The rigid endoscope 1 can properly use fixing of the bending portion 3 according to necessity to fix the entire bending portion 3 or the proximal end side of the bending portion 3 according to necessity. Since only the distal end portion of the bending portion 3 can be bent, the rigid endoscope 1 can be used to expand a tissue on the proximal end side of the bending portion 3, bend only the distal end portion, and observe an inside of a conduit.

### (Second Embodiment)

Next, a second embodiment of the present invention is explained. Note that, in the following explanation, concerning common components explained in the first embodiment, detailed explanation of the components is omitted using the same reference numerals and signs.

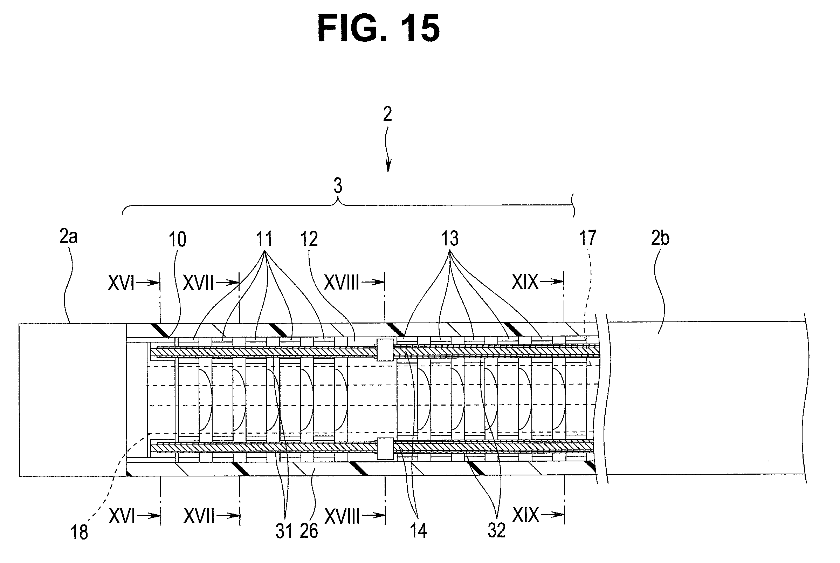

As shown in Fig. 15, the rigid endoscope 1 in the present embodiment is in a state in which, from the bending portion 3 of the insertion section 2 to the inside of the operation section 4, at least two or more bending operation wires 14 are inserted through bending-portion-entire-fixing elastic pipes 31, which are one kind of the traction member for bending fixing. Distal end portions of the respective bending operation wires 14 are fixedly connected to the wire fixing sections 10a provided in the bending-tube first joint ring 10.

Distal end portions of the bending-portion-entire-fixing elastic pipes 31 are also fixedly connected to the wire fixing sections 10a provided in the bending-tube first joint ring 10 in the same positions as the bending operation wires 14. Note that the bending operation wires 14 and the bending-portion-entire-fixing elastic pipes 31 are desirably disposed in opposed positions but do not always have to be disposed in the opposed positions.

As the bending-portion-entire-fixing elastic pipes 31, a material that is flexible, has elasticity, and less easily stretches in a longitudinal direction is used. The bending-portion-entire-fixing elastic pipes 31 are desirably made of a metal material having elasticity. However, other materials that are flexible, have elasticity, and less easily stretch in the longitudinal direction may be used.

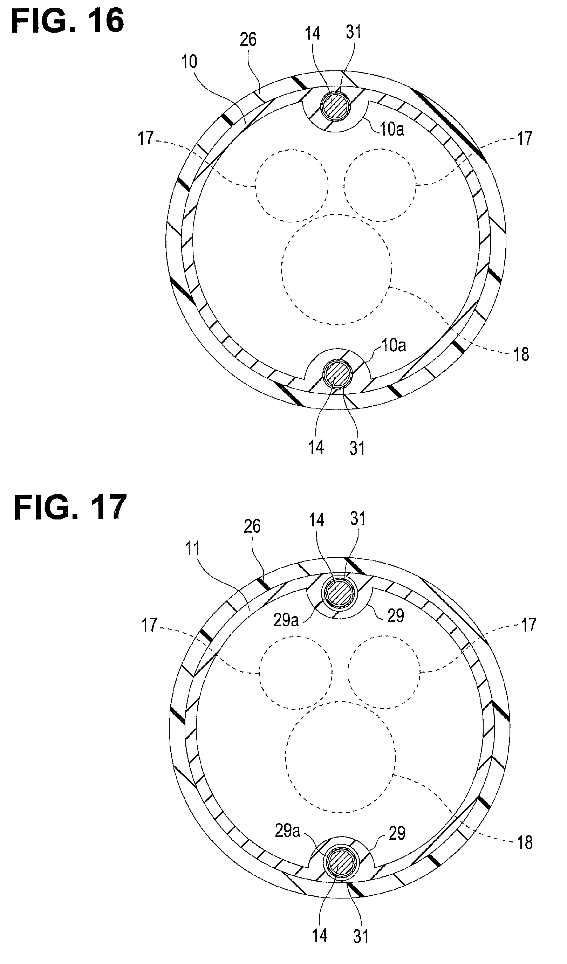

The bending operation wires 14 inserted through the bending-portion-entire-fixing elastic pipes 31 are inserted through the wire guide routes 29a formed in the wire guides 29 of the distal-end-side joint ring group 11, as shown in Fig. 17.

In the bending operation wires 14 and the bending-portion-entire-fixing elastic pipes 31, the proximal-end-side-joint-ring-group-fixing elastic pipes 32, which are one kind of the traction member for bending fixing, further cover outer sides of the bending-portion-entire-fixing elastic pipes 31 from an inside of the joint ring for relay 12 to the inside of the operation section 4.

Like the bending-portion-entire-fixing elastic pipes 31, as the proximal-end-side-joint-ring-group-fixing elastic pipes 32, a material that is flexible, has elasticity, and less easily stretches in a longitudinal direction is used. Like the bending-portion-entire-fixing elastic pipes 31, the proximal-end-side-joint-ring-group-fixing elastic pipes 32 are desirably made of a metal material having elasticity. However, other materials that are flexible, have elasticity, and less easily stretch in the longitudinal direction may be used.

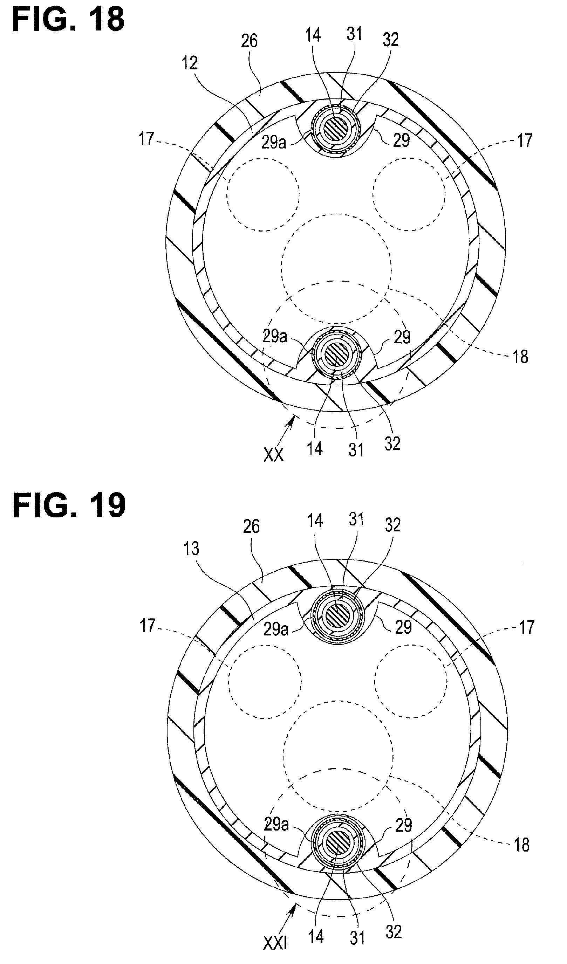

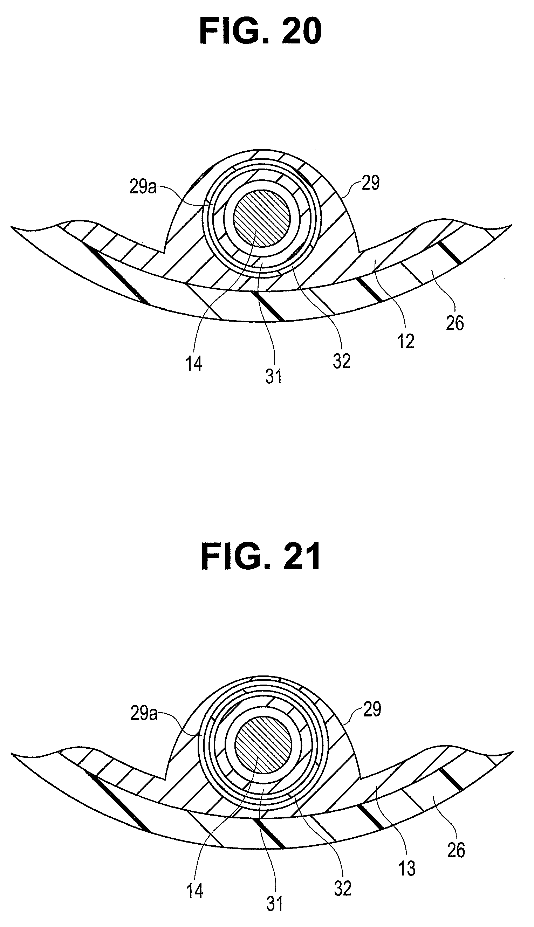

As shown in Fig. 18 and Fig. 20, distal end portions of the proximal-end-side-joint-ring-group-fixing elastic pipes 32 are fixedly connected to the wire guides 29 provided in the joint ring for relay 12. Note that, in the wire guides 29 of the joint ring for relay 12, the bending operation wires 14 and the bending-portion-entire-fixing elastic pipes 31 inserted through insides of the proximal-end-side-joint-ring-group-fixing elastic pipes 32 are not fixed as wires and pipes capable of advancing and retracting.

As shown in Fig. 19 and Fig. 21, from the joint ring for relay 12 to the operation section 4 side, a triple structure is formed in which the bending operation wires 14 are in a center, the bending-portion-entire-fixing elastic pipes 31 cover an outer side of the bending operation wires 14, and the proximal-end-side-joint-ring-group-fixing elastic pipes 32 cover an outer side of the bending-portion-entire-fixing elastic pipes 31.

The bending operation wires 14, the bending-portion-entire-fixing elastic pipes 31, and the proximal-end-side-joint-ring-group-fixing elastic pipes 32 pass, while keeping a state of the triple structure, the wire guide routes 29a formed in the wire guides 29 of the proximal-end-side joint ring group 13 and are inserted through the inside of the rigid tube 2b of the insertion section 2, and drawn into the operation section 4.

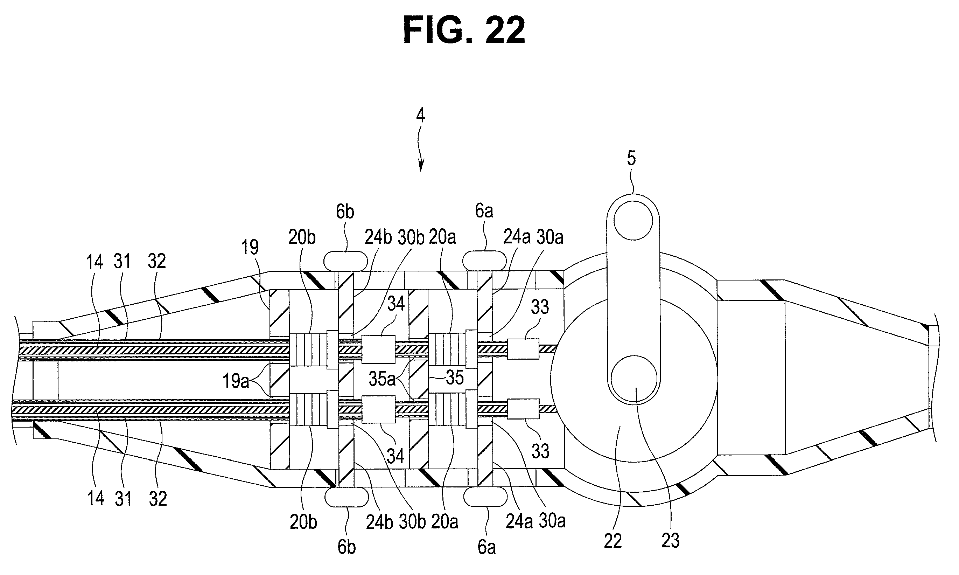

As shown in Fig. 22, on the inside of the operation section 4, as in the first embodiment, the bending fixing wire guide 19 is fixedly set on the inside. In the bending fixing wire guide 19, the holes 19a having size enough for allowing the proximal-end-side-joint-ring-group-fixing elastic pipes 32 covering the outer side of the bending-portion-entire-fixing elastic pipes 31, through which the bending operation wires 14 are inserted, to smoothly pass one by one are formed.

Note that the proximal-end-side-joint-ring-group-fixing elastic pipes 32 present on an outermost side of the triple structure, through which the bending operation wires 14 and the bending-portion-entire-fixing elastic pipes 31 are inserted through, are inserted through the holes 19a of the bending fixing wire guide 19 one by one. However, the proximal-end-side-joint-ring-group-fixing elastic pipes 32 may interfere with the holes 19a of the bending fixing wire guide 19.

The proximal-end-side-joint-ring-group-fixing elastic pipes 32, through which the bending operation wires 14 and the bending-portion-entire-fixing elastic pipes 31 are inserted through, are inserted through the springs for wire fixing 20b one by one after passing the holes 19a of the bending fixing wire guide 19.

One ends of the springs for wire fixing 20b are in contact with the bending fixing wire guide 19. Note that the contact ends of the springs for wire fixing 20b may be fixed to the bending fixing wire guide 19 or may be simply in contact with the bending fixing wire guide 19. An outer diameter of the springs for wire fixing 20b is set larger than a diameter of the holes 19a of the bending fixing wire guide 19.

The proximal-end-side-joint-ring-group-fixing elastic pipes 32 are subsequently inserted through the stopper release holes 30b formed in the proximal-end-side-joint-ring-group-fixing spring holders 24b one by one. Note that the stopper release holes 30b of the proximal-end-side-joint-ring-group-fixing spring holders 24b are set smaller than the outer diameter of the springs for wire fixing 20b.

Only the proximal-end-side-joint-ring-group-fixing elastic pipes 32 present on the outermost side of the triple structure, through which the bending operation wires 14 and the bending-portion-entire-fixing elastic pipes 31 passed through the stopper release holes 30b of the proximal-end-side-joint-ring-group-fixing spring holders 24b are inserted, are fixedly connected to the proximal-end-side-joint-ring-group-fixing elastic pipe stoppers 34.

Note that the bending operation wires 14 and the bending-portion-entire-fixing elastic pipes 31, through which the bending operation wires 14 are inserted, are inserted through the proximal-end-side-joint-ring-group-fixing elastic pipe stoppers 34.

An inner diameter of the proximal-end-side-joint-ring-group-fixing elastic pipe stoppers 34 is set larger than an outer diameter of the bending-portion-entire-fixing elastic pipes 31 such that the bending-portion-entire-fixing elastic pipes 31, through which the bending operation wires 14 are inserted, can smoothly pass. An outer diameter of the proximal-end-side-joint-ring-group-fixing elastic pipe stoppers 34 is set smaller than the hole diameter of the stopper release holes 30b of the proximal-end-side-joint-ring-group-fixing spring holders 24b.

The springs for wire fixing 20b described above are set to a dimension in a longitudinal direction with which, when distal ends of the springs for wire fixing 20b come into contact with a reference surface of the bending fixing wire guide 19 and the springs for wire fixing 20b are released to an free length on the basis of a position of the contact, the springs for wire fixing 20b come into contact with the proximal-end-side-joint-ring-group-fixing elastic pipe stoppers 34 and can push the proximal-end-side-joint-ring-group-fixing elastic pipe stoppers 34 with a predetermined force.

That is, the springs for wire fixing 20b are set to an urging force with which, when the distal ends of the springs for wire fixing 20b come into contact with a reference surface of the bending fixing wire guide 19 and the springs for wire fixing 20b are released to an free length on the basis of a position of the contact, the springs for wire fixing 20b come into contact with the proximal-end-side-joint-ring-group-fixing elastic pipe stoppers 34 and urge, with a predetermined force, the proximal-end-side-joint-ring-group-fixing elastic pipe stoppers 34 to a position where the proximal-end-side-joint-ring-group-fixing elastic pipes 32 connected to the proximal-end-side-joint-ring-group-fixing elastic pipe stoppers 34 receive tension to the proximal end side.

Note that an outer diameter of the proximal-end-side-joint-ring-group-fixing elastic pipe stoppers 34 is set larger than the inner diameter of the springs for wire fixing 20b.

The bending-portion-entire-fixing elastic pipes 31 covering the outer side of the bending operation wires 14 as a double structure, which pass the proximal-end-side-joint-ring-group-fixing elastic pipe stoppers 34, are inserted through the springs for wire fixing 20a one by one after passing holes 35a formed in a bending-portion-entire-fixing elastic pipe guide 35.

One ends of the springs for wire fixing 20a are in contact with the bending-portion-entire-fixing elastic pipe guide 35. The contact ends of the springs for wire fixing 20a may be fixed to the bending-portion-entire-fixing elastic pipe guide 35 or may be simply in contact with bending-portion-entire-fixing elastic pipe guide 35.

The outer diameter of the springs for wire fixing 20a is set larger than a hole diameter of the holes 35a of the bending-portion-entire-fixing elastic pipe guide 35.

The bending-portion-entire-fixing elastic pipes 31 covering the outer side of the bending operation wires 14 are further inserted through the stopper release holes 30a formed in the entire-bending-fixing spring holders 24a one by one after being inserted through the springs for wire fixing 20a. Note that the hole diameter of the stopper release holes 30a of the entire-bending-fixing spring holders 24a is set smaller than the outer diameter of the springs for wire fixing 20a.

The bending-portion-entire-fixing elastic pipes 31 inserted through the stopper release holes 30a are fixedly connected to bending-portion-entire-fixing elastic pipe stoppers 33. An inner diameter of the bending-portion-entire-fixing elastic pipe stoppers 33 is set larger than the outer diameter of the bending-portion-entire-fixing elastic pipes 31.

The bending operation wires 14 can smoothly pass insides of the bending-portion-entire-fixing elastic pipe stoppers 33. Note that an outer diameter of the bending-portion-entire-fixing elastic pipe stoppers 33 is set smaller than the stopper release holes 30a of the entire-bending-fixing spring holders 24a and set larger than the inner diameter of the springs for wire fixing 20a.

The springs for wire fixing 20a are set to a dimension in a longitudinal direction with which, when the distal ends of the springs for wire fixing 20a come into contact with a proximal end face of the bending-portion-entire-fixing elastic pipe guide 35 and the springs for wire fixing 20a are released to an free length on the basis of a position of the contact, the springs for wire fixing 20a come into contact with the bending-portion-entire-fixing elastic pipe stoppers 33 and can push the bending-portion-entire-fixing elastic pipe stoppers 33 to the proximal end side with a predetermined force.

That is, the springs for wire fixing 20a are set to an urging force with which, when the distal ends of the springs for wire fixing 20a come into contact with the bending-portion-entire-fixing elastic pipe guide 35 and the springs for wire fixing 20a are released to an free length on the basis of a position of the contact, the springs for wire fixing 20a come into contact with the bending-portion-entire-fixing elastic pipe stoppers 33 and urge, with a predetermined force, the bending-portion-entire-fixing elastic pipe stoppers 33 to a position where the bending-portion-entire-fixing elastic pipes 31 connected to the bending-portion-entire-fixing elastic pipe stoppers 33 receive tension to the proximal end side.

The proximal-end-side-joint-ring-group-fixing spring holders 24b are fixedly connected to the proximal-end-side-joint-ring-group fixing knobs 6b located on the outer surface of the operation section 4. By moving the proximal-end-side-joint-ring-group fixing knobs 6b, positions of the proximal-end-side-joint-ring-group-fixing spring holders 24b on the inside of the operation section 4 can be operated back and force from the outside.

By operating the proximal-end-side-joint-ring-group fixing knobs 6b, it is possible to move the proximal-end-side-joint-ring-group-fixing spring holders 24b connected to the proximal-end-side-joint-ring-group fixing knobs 6b to the distal end side and compress the springs for wire fixing 20b. Note that, in the operation section 4, as in the first embodiment, a not-shown fixing mechanism for locking the proximal-end-side-joint-ring-group fixing knobs 6b in a position where the springs for wire fixing 20b are compressed is provided.

The entire-bending-fixing spring holders 24a are fixedly connected to the entire-bending fixing knobs 6a located on an outer surface of the operation section 4. By moving the entire-bending fixing knobs 6a back and forth, positions of the entire-bending-fixing spring holders 24a on the inside of the operation section 4 can be operated back and force from the outside.

By operating the entire-bending fixing knobs 6a, it is possible to move the entire-bending-fixing spring holders 24a connected to the entire-bending fixing knobs 6a and compress the springs for wire fixing 20a. Note that, in the operation section 4, as in the first embodiment, a not-shown fixing mechanism for locking the entire-bending fixing knobs 6a in a position where the springs for wire fixing 20a are compressed is provided.

In the rigid endoscope 1 in the present embodiment configured as explained above, for example, as a state shown in Fig. 22, in a state in which both of the entire-bending fixing knobs 6a and the proximal-end-side-joint-ring-group fixing knobs 6b are present in positions on the distal end side, the entire-bending-fixing spring holders 24a and the proximal-end-side-joint-ring-group-fixing spring holders 24b coupled to the entire-bending fixing knobs 6a and the proximal-end-side-joint-ring-group fixing knobs 6b compress all of the springs for wire fixing 20a and 20b.

The wire fixing springs 20a and 20b do not come into contact with the bending-portion-entire-fixing elastic pipe stoppers 33 and the proximal-end-side-joint-ring-group-fixing elastic pipe stoppers 34 in this state. The bending-portion-entire-fixing elastic pipe stoppers 33 and the proximal-end-side-joint-ring-group-fixing elastic pipe stoppers 34 can freely move back and forth.

At this point, the bending operation wires 14 inserted through the bending-portion-entire-fixing elastic pipe stoppers 33 and the proximal-end-side-joint-ring-group-fixing elastic pipe stoppers 34 can freely move back and forth.

That is, as shown in Fig. 22, in an operation position where the entire-bending fixing knobs 6a and the proximal-end-side-joint-ring-group fixing knobs 6b move to the distal end side, both of the bending-portion-entire-fixing elastic pipes 31, to which the bending-portion-entire-fixing elastic pipe stoppers 33 are connected, and the proximal-end-side-joint-ring-group-fixing elastic pipes 32, to which the proximal-end-side-joint-ring-group-fixing elastic pipe stoppers 34 are connected, can freely move back and forth.

Therefore, the bending-tube first joint ring 10, the distal-end-side joint ring group 11, the joint ring for relay 12, and the proximal-end-side joint ring group 13 provided in the bending portion 3 do not receive a traction force to the proximal end side from the bending-portion entire fixing wires 15 and the proximal-end-side-joint-ring-group fixing wires 16. The bending-portion entire fixing wires 15 and the proximal-end-side-joint-ring-group fixing wires 16 can freely turn in the respective coupling parts.

Consequently, by operating the bending operation lever 5, it is possible to freely perform bending operation of the bending portion 3 according to pulling and loosening of the pair of bending operation wires 14 coupled to the drum unit 22 associated with the bending operation lever 5.

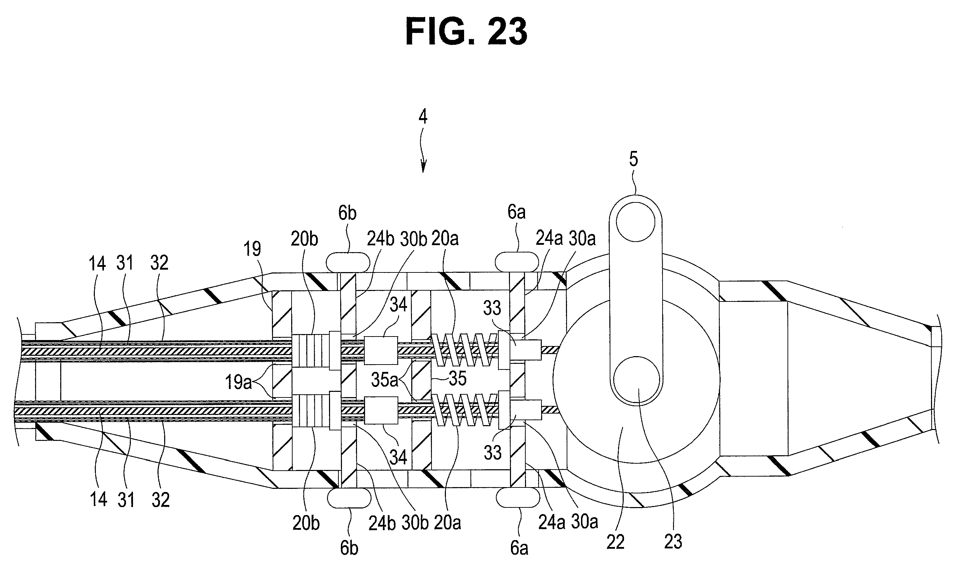

As shown in Fig. 23, for example, in an operation position where the entire-bending fixing knobs 6a move to the proximal end side and the proximal-end-side-joint-ring-group fixing knobs 6b move to the distal end side, only the entire-bending-fixing spring holders 24a coupled to the entire-bending fixing knobs 6a do not compress but release the springs for wire fixing 20a. At this point, the bending-portion-entire-fixing elastic pipe stoppers 33 pass the stopper release holes 30a provided in the entire-bending-fixing spring holders 24a.

One ends on the distal end side of the springs for wire fixing 20a, the compression of which is released, press the bending-portion-entire-fixing elastic pipe guide 35. However, since the bending-portion-entire-fixing elastic pipe guide 35 is fixed and provided on the inside of the operation section 4, the springs for wire fixing 20a press the bending-portion-entire-fixing elastic pipe stoppers 33 in contact with the springs for wire fixing 20a to the proximal end side.

Consequently, all of the bending-portion-entire-fixing elastic pipes 31, to which the bending-portion-entire-fixing elastic pipe stoppers 33 are connected, are attracted and pulled to the proximal end side by an urging force of the springs for wire fixing 20a. That is, the springs for wire fixing 20a configure a traction member for bending portion fixing that pulls the bending-portion-entire-fixing elastic pipes 31.

In this state, the bending-tube first joint ring 10, the distal-end-side joint ring group 11, the joint ring for relay 12, and the proximal-end-side joint ring group 13 receive a traction force to the proximal end side by the bending-portion-entire-fixing elastic pipes 31. All of the bending-tube first joint ring 10, the distal-end-side joint ring group 11, the joint ring for relay 12, and the proximal-end-side joint ring group 13 are drawn to the proximal end side.

That is, the bending-tube first joint ring 10, the distal-end-side joint ring group 11, the joint ring for relay 12, and the proximal-end-side joint ring group 13 are compressed to the proximal end side. Mobility in the respective coupling parts is suppressed. As a result, movement of the pair of bending operation wires 14 is also suppressed. The bending operation lever 5 that pulls and loosens the bending operation wires 14 cannot be freely moved, operation of the bending operation lever 5 is suppressed, and bending operation of the bending portion 3 cannot be freely performed.

Note that, at this point, as in the first embodiment, the bending portion 3 is in a fixed state in which the bending portion 3 is substantially linear and not bent. That is, the insertion section 2 is fixed in a substantially linear shape. However, when a force exceeding the urging force of the springs for wire fixing 20a is directly applied to the bending portion 3 from the outside, the bending portion 3 can bend.

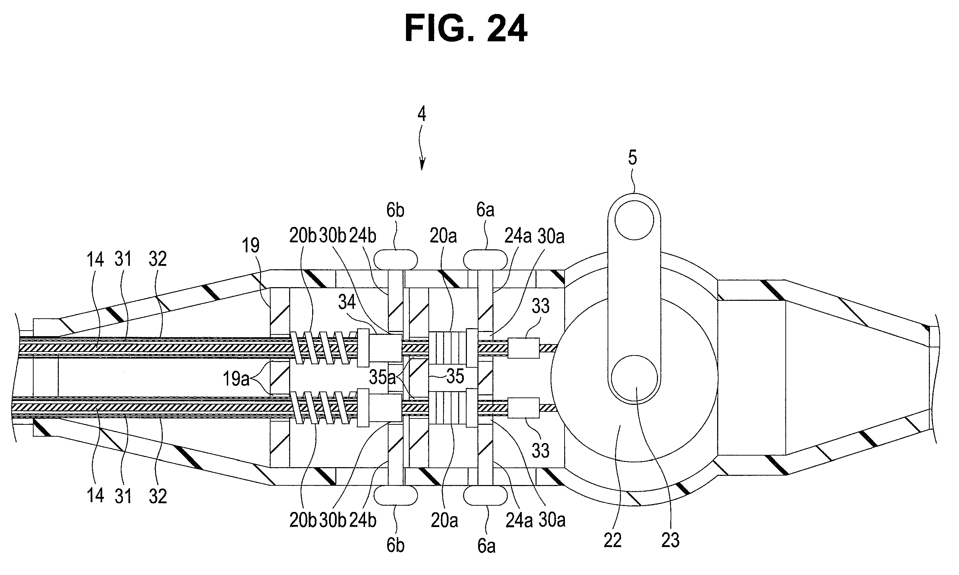

As shown in Fig. 24, for example, in an operation position where the entire-bending fixing knobs 6a are moved to the distal end side and the proximal-end-side-joint-ring-group fixing knobs 6b are moved to the proximal end side, only the proximal-end-side-joint-ring-group-fixing spring holders 24b coupled to the proximal-end-side-joint-ring-group fixing knobs 6b release the compression of the springs for wire fixing 20b. At this point, the proximal-end-side-joint-ring-group-fixing elastic pipe stoppers 34 pass the stopper release holes 30b provided in the proximal-end-side-joint-ring-group-fixing spring holders 24b.

As explained above, one ends on the distal end side of the springs for wire fixing 20b, the compression of which is released, press the bending fixing wire guide 19 and press the proximal-end-side-joint-ring-group-fixing elastic pipe stoppers 34 in contact with the springs for wire fixing 20b to the proximal end side.

Consequently, all of the proximal-end-side-joint-ring-group-fixing elastic pipes 32, to which the proximal-end-side-joint-ring-group-fixing elastic pipe stoppers 34 are coupled, are attracted and pulled to the proximal end side by an urging force of the springs for wire fixing 20b. That is, the springs for wire fixing 20b configure a traction member for bending portion fixing that pulls the proximal-end-side-joint-ring-group-fixing elastic pipes 32.

In this state, only the joint ring for relay 12, to which distal ends of the proximal-end-side-joint-ring-group-fixing elastic pipes 32 are fixed, and the proximal-end-side joint ring group 13 on the proximal end side of the joint ring for relay 12 receive a traction force to the proximal end side by the proximal-end-side-joint-ring-group-fixing elastic pipes 32 and drawn to the proximal end side.

That is, the joint ring for relay 12 and the proximal-end-side joint ring group 13 are compressed to the proximal end side. Mobility in the respective coupling parts is suppressed. As in the first embodiment, the bending portion 3 here is in a fixed state in which only the part where the joint ring for relay 12 and the proximal end side joint ring group 13 are provided is substantially linear and not bent.

In the bending-tube first joint ring 10 and the distal-end-side joint ring group 11 that do not receive the traction force to the proximal end side by the proximal-end-side-joint-ring-group-fixing elastic pipes 32, mobility in the respective coupling parts is not suppressed. The bending-tube first joint ring 10 and the distal-end-side joint ring group 11 can turn freely.

Therefore, by operating the bending operation lever 5, it is possible to perform bending operation of a part where the bending-tube first joint ring 10 and the distal-end-side joint ring group 11 are provided on the distal end side of the bending portion 3.

Note that, as explained above, when a force exceeding the urging force of the springs for wire fixing 20b is directly applied from the outside to the part where the joint ring for relay 12 and the proximal-end-side joint ring group 13 are provided on the proximal end side of the bending portion 3 or when the force is applied to the bending operation lever 5, the entire bending portion 3 can bend.

As explained above, as in the first embodiment, the rigid endoscope 1 in the present embodiment is configured to be capable of selectively taking, by operating back and forth the entire-bending fixing knobs 6a and the proximal-end-side-joint-ring-group fixing knobs 6b provided in the operation section 4, a state in which bending operation of the bending portion 3 can be freely performed, a state in which the bending portion 3 can be substantially linearly fixed, or a state in which bending operation of only the distal end side of the bending portion 3 can be performed and the proximal end side can be substantially linearly fixed.

That is, the rigid endoscope 1 can be configured to be capable of selecting preservation of a bending function only in a part of the bending portion 3, that is, the proximal end side while hardening the entire insertion section 2 or hardening a part of the bending portion 3.

By adopting such a configuration, in the rigid endoscope 1 in the present embodiment, in addition to the action and effects described in the first embodiment, since the operation wires 14, the bending-portion-entire-fixing elastic pipes 31, and the proximal-end-side-joint-ring-group-fixing elastic pipes 32 are formed in a triple structure, a space for inserting the operation wires 14, the bending-portion-entire-fixing elastic pipes 31, and the proximal-end-side-joint-ring-group-fixing elastic pipes 32 through the insertion section 2 decreases. Therefore, it is possible to reduce a diameter of the insertion section 2.

Note that, as the examples explained in the present invention, the configuration of the bending portion 3 in which the plurality of joint rings 10, 11, 12, and 13 are turnably coupled by the rivets 25 and the like is explained as examples. However, a basic configuration of the bending portion 3 is not limited to these examples. The bending portion 3 may be any form as long as the bending portion 3 can be bent by being pulled by the bending operation wires 14.

The invention described in the embodiments described above is not limited to the embodiments and the modifications. Besides, in an implementation stage, various variations can be carried out in a range not departing from the spirit of the invention. Further, the embodiments include inventions in various stages. Various inventions can be extracted according to appropriate combinations of a disclosed plurality of constituent elements.

For example, when the described problems can be solved and the described effects can be obtained even if several constituent elements are deleted from all the constituent elements described in the embodiments, a configuration in which the constituent elements are deleted can be extracted as an invention.

This application is based upon and claims the benefit of priority from Japanese Patent Application No. 2013-113228 filed in Japan on May 29, 2013, the contents of which are incorporated in the specification, the claims, and the drawings of Japanese Patent Application No. 2013-113228.

## Claims

1. An endoscope comprising:
an insertion section including a distal end portion and a bending portion;
a traction member for bending driving connected to the bending portion and inserted through the insertion section;
a bending operation section that performs pulling and loosening operation of the traction member for bending driving and performs bending operation of the entire bending portion;
a first traction member for bending fixing connected to the bending portion and inserted through the insertion section;
a first operation section for bending fixing that performs pulling and loosening operation of the first traction member for bending fixing and operates fixing or release of the entire bending portion;
a second traction member for bending fixing connected to the bending portion and inserted through the insertion section; and
a second operation section for bending fixing that performs pulling and loosening operation of the second traction member for bending fixing and operates fixing or release of a part of the bending portion.

2. The endoscope according to claim 1, wherein a distal end of the second traction member for bending portion fixing is fixed to a predetermined position of the bending portion, the bending portion further on a proximal end side than a fixing position of the second traction member for bending portion fixing can be fixed by pulling operation by the second operation section for bending fixing, and only the bending portion further on a distal end side than the fixing position of the second traction member for bending portion fixing can be bent by pulling operation by the bending operation section.

3. The endoscope according to claim 1 or 2, wherein the traction member for bending driving, the first traction member for bending fixing, and the second traction member for bending fixing are formed of wires.

4. The endoscope according to claim 1 or 2, wherein the traction member for bending driving is configured by a wire, the first traction member for bending fixing is configured by a tubular member, and the traction member for bending driving is disposed on an inside of the first traction member for bending fixing.

5. The endoscope according to claim 4, wherein the second traction member for bending fixing is configured by a tubular member, and the first traction member for bending fixing is disposed on an inside of the second traction member for bending fixing.

6. The endoscope according to any one of claims 1 to 5, wherein a distal end of the second traction member for bending fixing is fixed in a position shifted to a proximal end side in a longitudinal direction of the bending portion with respect to the first traction member for bending fixing, and a fixed range and a bendable range of the bending portion are made selectably operable by the first operation section for bending fixing and the second operation section for bending fixing.
